(19) Europäisches Patentamt
European Patent Office
Office européen des brevets

(11) Veröffentlichungsnummer: **0 546 474 A1**

(12) **EUROPÄISCHE PATENTANMELDUNG**

(21) Anmeldenummer: **92120724.7**

(22) Anmeldetag: **04.12.92**

(51) Int. Cl.5: **C07D 491/107**, C12P 17/18, A61K 31/40, //(C07D491/107, 307:00,209:00),(C12P17/18, C12R1:68)

(30) Priorität: **07.12.91 DE 4140382**

(43) Veröffentlichungstag der Anmeldung: **16.06.93 Patentblatt 93/24**

(84) Benannte Vertragsstaaten: **AT BE CH DE DK ES FR GB GR IT LI LU MC NL PT SE**

(71) Anmelder: **HOECHST AKTIENGESELLSCHAFT**
**Postfach 80 03 20**
**W-6230 Frankfurt am Main 80(DE)**

(72) Erfinder: **Wink, Joachim, Dr.**
**Magdeburger Strasse 14**
**W-6074 Rödermark(DE)**
Erfinder: **Grabley, Susanne, Dr.**
**Hölderlinstrasse 7**
**W-6240 Königstein/Ts.(DE)**
Erfinder: **Gareis, Manfred, Dr.**
**Hildachstrasse 11c**
**W-8000 München(DE)**
Erfinder: **Thiericke, Ralf, Dr.**
**Drosselweg 3**
**W-6230 Frankfurt am Main 80(DE)**
Erfinder: **Kirsch, Reinhard, Dr.**
**Johannesallee 18**
**W-6230 Frankfurt am Main 80(DE)**

(54) **Pseurotin F1/F2, neue Metabolite aus Aspergillus fumigatus, Verfahren zu ihrer Herstellung und ihre Verwendung als Apomorphin Antagonisten.**

(57) Mit Hilfe von Aspergillus fumigatus werden die neuen Metabolite der folgenden Formeln hergestellt

EP 0 546 474 A1

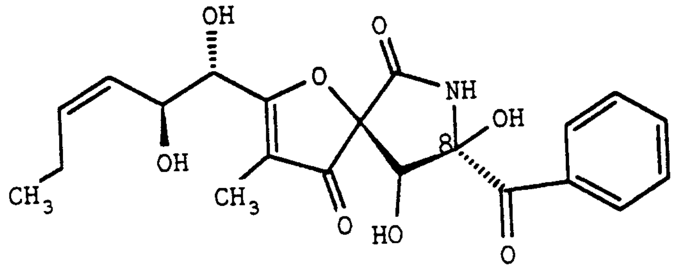

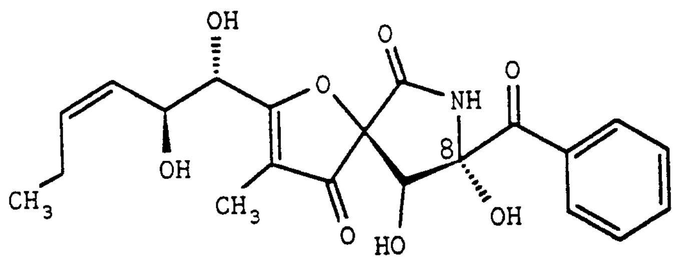

Es handelt sich bei diesen Metaboliten um Pseurotin $F_1/F_2$, die sich aufgrund ihrer Stereochemie am C-8-Atom unterscheiden.

Die Metabolite sind insbesondere als Apomorphin Antagonisten einzusetzen.

Sekundärmetabolite aus Pilzen sind schon seit langer Zeit bekannt und werden im großen Maßstab therapeutisch angewandt. Die Pseurotine (A - E) sind als biologisch nicht aktive Metabolite des Ascomyceten Pseudeurotium ovalis beschrieben (Fungal Metabolite 2, W.B. Turner, Academic Press 1983). Kürzlich wurde Synerazol aus Aspergillus fumigatus Stamm SANK 10588 (O. Ando et al., J. Antibiotics 44, 383, 1991) beschrieben. Dieser Metabolit ist ebenfalls strukturell in die Gruppe der Pseurotine einzuordnen und besitzt antifungische Aktivität. Außerdem wurde aus Aspergillus fumigatus Fresenius eine Substanz ($C_{22}$ $H_{22}$ $NO_6$) mit Antitumorwirkung beschrieben (Mizogami et al., JP 03 10, 689).

Es wurde nun gefunden, daß Aspergillus fumigatus neue Metabolite, die Pseurotine $F_1/F_2$, produziert. Die beiden Metabolite unterscheiden sich hinsichtlich ihrer Formel durch eine entgegengesetzte Stereochemie am C-8-Atom und entstehen während der Fermentation des Mikroorganismus als Diastereomere. Pseurotin $F_1/F_2$ besitzen pharmakologische und damit therapeutische Wirksamkeit und können vorteilhaft als Apomorphin Antagonisten mit entsprechendem pharmakologischen Nutzen eingesetzt werden.

Die Erfindung betrifft somit:

1. Die Verbindungen der Formeln

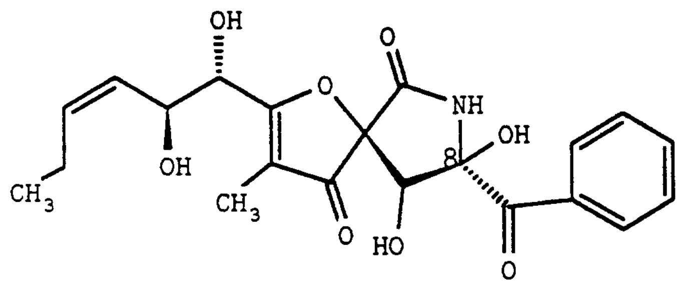

Ia

Pseurotin F1

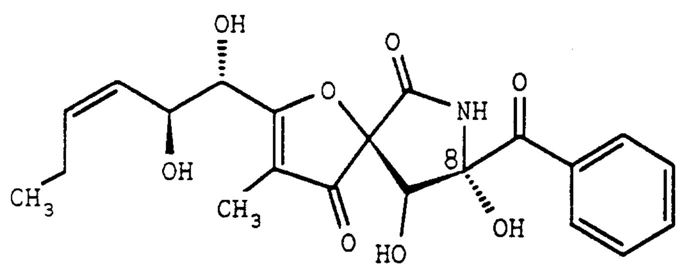

I b

Pseurotin F2

2. Ein Verfahren zur Herstellung der Verbindungen der Formeln Ia und Ib, das dadurch gekennzeichnet ist, daß Aspergillus fumigatus in einem Nährmedium kultiviert wird, bis sich die Verbindungen der Formeln Ia und Ib in der Kultur anhäufen.

3. Eine Verwendung der Verbindungen der Formeln Ia und Ib als pharmakologisch wirksame Substanzen, insbesondere als Apomorphin-Antagonisten.

Im folgenden wird die Erfindung detailliert beschrieben, insbesondere in ihren bevorzugten Ausführungsformen. Ferner wird die Erfindung durch den Inhalt der Patentansprüche bestimmt.

Die erfindungsgemäßen Verbindungen werden mit Aspergillus fumigatus, bevorzugt mit Aspergillus fumigatus DSM 6598 hergestellt. Bei der Fermentation von Aspergillus fumigatus entstehen die Verbindungen der Formeln Ia und Ib als ein Pseurotin-Gemisch aus Pseurotin $F_1/F_2$ im Verhältnis 4:1. Die beiden Verbindungen unterscheiden sich durch ihre Stereochemie am C-8-Atom.

Der Stamm wurde von einer Hautprobe einer Katze isoliert. Aspergillus fumigatus DSM 6598 wurde bei der Deutschen Sammlung von Mikroorganismen und Zellkulturen GmbH, Mascheroder Weg 1b, 3300 Braunschweig, Deutschland, nach den Regeln des Budapester Vertrages am 05.07.1991 unter oben genannter Nummer hinterlegt.

Anstelle des Stammes DSM 6598 können auch dessen Mutanten und Varianten eingesetzt werden, soweit sie die Verbindungen der Formeln Ia und Ib synthetisieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS), 2-Hydroxy-4-methoxybenzophenon (MOB) oder N-Methyl-N'-nitro-N-nitrosoguanidin (MNNG) erzeugt werden.

Das Screening nach Mutanten und Varianten, die die Verbindungen der Formeln Ia und Ib synthetisieren, erfolgt nach folgendem Schema:

- Abtrennung des Mycels nach der Fermentation;
- Adsorption des Kulturfiltrates an ein Polystyroladsorberharz;
- Elution mit 50 %igem Methanol;
- Aufkonzentrierung und Analytik mittels Dünnschichtchromatographie auf Kieselgelplatten; als Laufmittel wird ein Chloroform-Methanol-Gemisch (Verhältnis 9:1) verwendet;
- Detektion mittels Ansprühen mit Tetrazoliumblau (z. B. Fa. Merck, Darmstadt, Deutschland).

Aspergillus fumigatus DSM 6598 besitzt weißes Luftmycel und grüne Conidien. Er bildet die für Aspergillus fumigatus charakteristischen Condidiophoren mit langen parallelen Conidien-Ketten.
In einer Nährlösung, die eine Kohlenstoffquelle und eine Stickstoffquelle sowie die üblichen anorganischen Salze enthält, produziert Aspergillus fumigatus die Verbindungen der allgemeinen Formeln Ia und Ib. Aspergillus fumigatus DSM 6598 produziert die Verbindungen der allgemeinen Formeln Ia und Ib in besonders großen Mengen.

Als bevorzugte Kohlenstoffquellen für die aerobe Fermentation eignen sich assimilierbare Kohlenhydrate und Zuckeralkohole, wie Glukose, Laktose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der Verbindungen der Formeln Ia und Ib verläuft besonders gut in einer Nährlösung, die etwa 0,2 bis 5 %, bevorzugt 1 bis 4 %, Malzextrakt und 0,02 bis 0,5 % bevorzugt 0,1 bis 0,4 % Hefeextrakt enthält, sowie 0,2 bis 5,0 %, bevorzugt 0,5 bis 2 % Glucose und 0,01 bis 0,1 %, bevorzugt 0,04 bis 0,06 % $(NH_4)_2HPO_4$, jeweils bezogen auf das Gewicht der gesamten Nährlösung. Die Kultivierung erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 15 bis 30 ° C, vorzugsweise bei etwa 20 bis 30 ° C, insbesondere bei 23 bis 28 ° C durchgeführt werden. Der pH-Bereich sollte zwischen 1 und 7 liegen, vorteilhaft zwischen 2,5 und 4,5. Man kultiviert den Mikroorganismus unter diesen Bedingungen im allgemeinen über einen Zeitraum von 24 bis 300 Stunden, bevorzugt 36 bis 140 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z.B., indem man ein versportes Mycel in eine Nährlösung überimpft und etwa 36 bis 120 Strunden, bevorzugt 48 bis 72 Stunden, wachsen läßt. Das versporte Mycel kann beispielsweise erhalten werden, indem man den Stamm etwa 3 bis 40 Tage, bevorzugt 4 bis 10 Tage, auf einem festen oder flüssigen Nährboden, beispielsweise Hefe-Malz-Agar oder Kartoffel-Dextrose-Agar, wachsen läßt.

Der Fermentationsverlauf kann anhand des pH-Wertes der Kultur oder des Mycelvolumens sowie durch chromatographische Methoden, wie z.B. Dünnschichtchromatographie oder High Pressure Liquid Chromatography, überwacht werden. Die Verbindungen der Formeln Ia und Ib sind sowohl im Mycel als auch im Kulturfiltrat enthalten.

Die Isolierung der genannten Verbindungen aus dem Kulturmedium erfolgt nach bekannten Methoden unter Berücksichtigung der chemischen, physikalischen und biologischen Eigenschaften der Produkte. Zum Testen der Metabolit-Konzentration im Kulturmedium oder in den einzelnen Isolierungsstufen kann die Dünnschichtchromatographie, beispielsweise an Kieselgel mit Butanol/Eisessig/Wasser oder Ethylacetat/Methanol/Wasser Mischungen als Laufmittel verwendet werden. Die Detektion bei der dünnschichtchromatographischen Auftrennung kann beispielsweise durch UV-Löschung und Färbereagentien wie

Anisaldehyd oder Tetrazolblau erfolgen, wobei die Menge der gebildeten Substanz zweckmäßig mit einer Eichlösung verglichen wird.

Zur Isolierung der Verbindungen der Formeln Ia und Ib werden Kulturbrühe und Mycel zuerst z.B. durch Filtration oder Zentrifugation getrennt. Das Kulturfiltrat wird über ein Adsoberharz wie z.B. XAD Adsorber der Fa. Amberlite gegeben, an dem die Verbindungen der Formel I binden und mit einem Lösungsmittel/Wasser-Gemisch, z.B. Methanol Wasser 4:1, eluiert werden können.

Die Auftrennung des Gemisches Pseurotin $F_1/F_2$ erfolgt dünnschichtchromatographish mit dem Laufmittelgemisch Ethylacetat-H/Heptan (2:1).

Die Reinisolierung der Verbindungen der Formeln Ia und Ib erfolgt durch Chromatographie an geeigneten Materialien, vorzugsweise z.B. an Kieselgel, Aluminiumoxid, Ionenaustauschern oder Adsorberharzen, durch anschließende Elution mit organischen, polaren Lösungsmitteln oder Lösungsmittelgemischen, wie z.B. Essigsäurealkylester, Gemische aus Essigsäurealkylester mit einem niederen Alkanol, Chloroform oder Methylenchlorid bzw. Gemischen dieser Lösungsmittel mit niederen Alkanolen, gegebenenfalls auch mit Wasser, bzw. einem für Ionenaustauscher-Harzen geeigneten pH- bzw. Salzgradienten, wie beispielsweise Kochsalz oder Tris-(hydroxymethyl)-aminomethan-HCl(Tris-Puffer), und Vereinigung der biologisch aktiven Fraktionen.

Die Verbindungen der Formeln Ia und Ib sind im festen Zustand und in Lösungen im pH-Bereich zwischen 3 und 8, insbesondere 5 und 7, stabil und lassen sich damit in übliche galenische Zubereitungen einarbeiten.

Die erfindungsgemäßen Verbindungen besitzen biologische Aktivität indem sie eine Wirkung als Apomorphin-Antagonisten zeigen.

Die Anwendung betrifft auch pharmazeutische Zubereitungen der Verbindungen der Formeln Ia und Ib. Biologisch aktives Pseurotin $F_1/F_2$ gehört in die Gruppe der Neuroleptika, d.h. der Psychopharmaka.

Bei der Herstellung von Arzneimitteln können neben dem Wirkstoff auch pharmazeutisch unbedenkliche Zusatzstoffe, wie Verdünnungsmittel und/oder Trägermaterialien, verwendet werden. Hierunter sind physiologisch unbedenkliche Substanzen zu verstehen, die nach Vermischen mit dem Wirkstoff diesen in eine für die Verabreichung geeignete Form bringen.

Geeignete feste oder flüssige galenische Zubereitungsformen sind beispielsweise Tabletten, Dragees, Pulver, Kapseln, Suppositorien, Sirupe, Emulsionen, Suspensionen, Tropfen oder injizierbare Lösungen sowie Präparate mit protrahierter Wirkstoff-Freigabe. Als häufig verwendete Trägerstoffe bzw. Verdünnungsmittel seien z.B. verschiedene Zucker oder Stärkearten, Cellulosederivate, Magnesiumcarbonat, Gelatine, tierische und pflanzliche Öle, Polyethylenglykole, Wasser oder andere geeignete Lösungsmittel sowie wasserhaltige Puffermittel, die durch Zusatz von Glucose oder Salzen isotonisch gemacht werden können, genannt.

Außerdem können gegebenenfalls oberflächenaktive Mittel, Farb- und Geschmacksstoffe, Stabilisatoren, sowie Konservierungsmittel als weitere Zusatzstoffe in den erfindungsgemäßen Arzneimittelnzubereitungen Verwendung finden. Es können auch pharmakologisch akzeptable polymere Träger, wie z.B. Polyphenylpyrolidone, oder andere pharmazeutisch akzeptable Additive, wie z.B. Cyclodextrin oder Polysaccharide, verwendet werden.

Die Präparate können oral, rektal oder parenteral verabreicht werden. Vorzugsweise können die Präparate in Dosierungseinheiten hergestellt werden; insbesondere Tabletten, Suppositorien, stellen Beispiele für geeignete Dosierungseinheiten dar.

Gegebenenfalls können die Dosierungseinheiten für die orale Verabreichung mikroverkapselt werden, um die Abgabe zu verzögern, oder über einen längeren Zeitraum auszudehnen, wie beispielsweise durch Überziehen oder Einbetten des Wirkstoffs in Teilchenform in geeignete Polymere, Wachse oder dergleichen.

Die parenterale Verabreichung kann unter Verwendung flüssiger Dosierungsformen, wie steriler Lösungen und Suspensionen, erfolgen, die für die intramuskuläre oder subcutane Injektion bestimmt sind. Derartige Dosierungsformen werden durch Lösen bzw. Suspendieren einer abgemessenen Wirkstoffmenge in einem geeigneten physiologisch unbedenklichen Verdünnungsmittel wie beispielsweise einem wäßrigen oder öligen Medium und Sterilisierung der Lösung bzw. der Suspension gegebenenfalls unter Mitverwendung von geeigneten Stabilisatoren, Emulgatoren und/oder Konservierungsmitteln und/oder Antioxidantien hergestellt.

Die pharmazeutischen Präparate werden nach allgemein üblichen Verfahren hergestellt. Das Dosierungsschema kann vom Typ, Alter, Gewicht, Geschlecht und medizinischen Zustand des Patienten abhängen.

In den folgenden Beispielen wird die Erfindung in weiteren Details beschrieben. Prozentangaben beziehen sich auf das Gewicht, wenn nicht anders angegeben.

Beispiele

1. a) Herstellung einer Sporensuspension des Produzentenstammes:

100 ml Nährlösung (20 g Malzextrakt, 2 g Hefeextrakt, 10 g Glucose, 0,5 g $(NH_4)_2HPO_4$ in 1 l Leitungswasser, pH-Wert vor der Sterilisation 6,0) in einem 500 ml sterilen Erlenmeyerkolben werden mit dem Stamm DSM 6598 beimpft und 72 Stunden bei 25 °C und 140 UpM auf einer rotierenden Schüttelmaschine inkubiert. Anschließend werden 20 ml Kulturflüssigkeit in einem sterilen 500 ml Erlenmeyerkolben mit dem Nährboden (Kartoffelinfus 4,0 g/l [Infus aus 200 g Kartoffeln in 1000 ml $H_2O$], 20,0 g/l D-Glucose, pH vor der Sterilisation 5,6), dem zusätzlich 15 g Agar/l zur Verfestigung zugegeben wurde, gleichmäßig verteilt und dekantiert. Die Kulturen werden 10 bis 14 Tage bei 25 °C inkubiert. Die nach dieser Zeit entstandenen Sporen eines Kolbens werden mit 500 ml entionisiertem Wasser, das einen Tropfen eines handelsüblichen nichtionischen Tensids (z.B. ®Triton X 100, Fa. Serva) enthält, abgeschwemmt, sofort weiterverwendet oder bei -22 °C in 50 % Glycerin aufbewahrt.

b) Herstellung einer Kultur bzw. einer Vorkultur des Produzentenstammes im Erlenmeyerkolben:

Ein steriler 500 ml Erlenmeyerkolben mit 100 ml der unter a) beschriebenen Nährlösung wird mit einer auf einem Schrägröhrchen gewachsenen Kultur oder mit 0,2 ml Sporensuspension angeimpft und auf einer Schüttelmaschine bei 140 UpM und 25 °C inkubiert. Die maximale Produktion der Verbindungen der Formel I ist nach ca. 120 Stunden erreicht. Zum Animpfen von 10 und 100 l Fermentern genügt eine 72 Stunden alte Submerskultur (Animpfmenge ca. 5 %) aus der gleichen Nährlösung.

2. Herstellung der Verbindungen der allgemeinen Formel I:

Ein 10 l Fermenter wird unter folgenden Bedingungen betrieben:
Nährmedium:

| | |
|---|---|
| 20 g/l | Malzextrakt |
| 10 g/l | Glucose |
| 2 g/l | Hefeextrakt |
| 0,5 g/l | $(NH_4)_2HPO_4$ |
| pH 6,0 | (vor der Sterilisation) |

| | |
|---|---|
| Inkubationszeit: | 120 Stunden |
| Inkubationstemperatur: | 25 °C |
| Rührergeschwindigkeit: | 200 UpM |
| Belüftung: | 5 l Luft/min. |

Durch wiederholte Zugabe weniger Tropfen ethanolischer Polyollösung kann die Schaumbildung unterdrückt werden. Das Produktionsmaximum wird nach ca. 96 Stunden erreicht. Der pH-Wert beträgt bei der Ernte ca. 3,5. Die Ausbeuten liegen bei ca. 10 mg/l.

3. Isolierung der Verbindungen der allgemeinen Formel I:

Nach Beendigung der Fermentation von DSM 6598 wird die Kulturbrühe unter Zusatz von ca. 2 % Filterhilfsmittel (z.B. ®Celite) filtriert. Es kann nach folgenden Schemata aufgearbeitet werden:

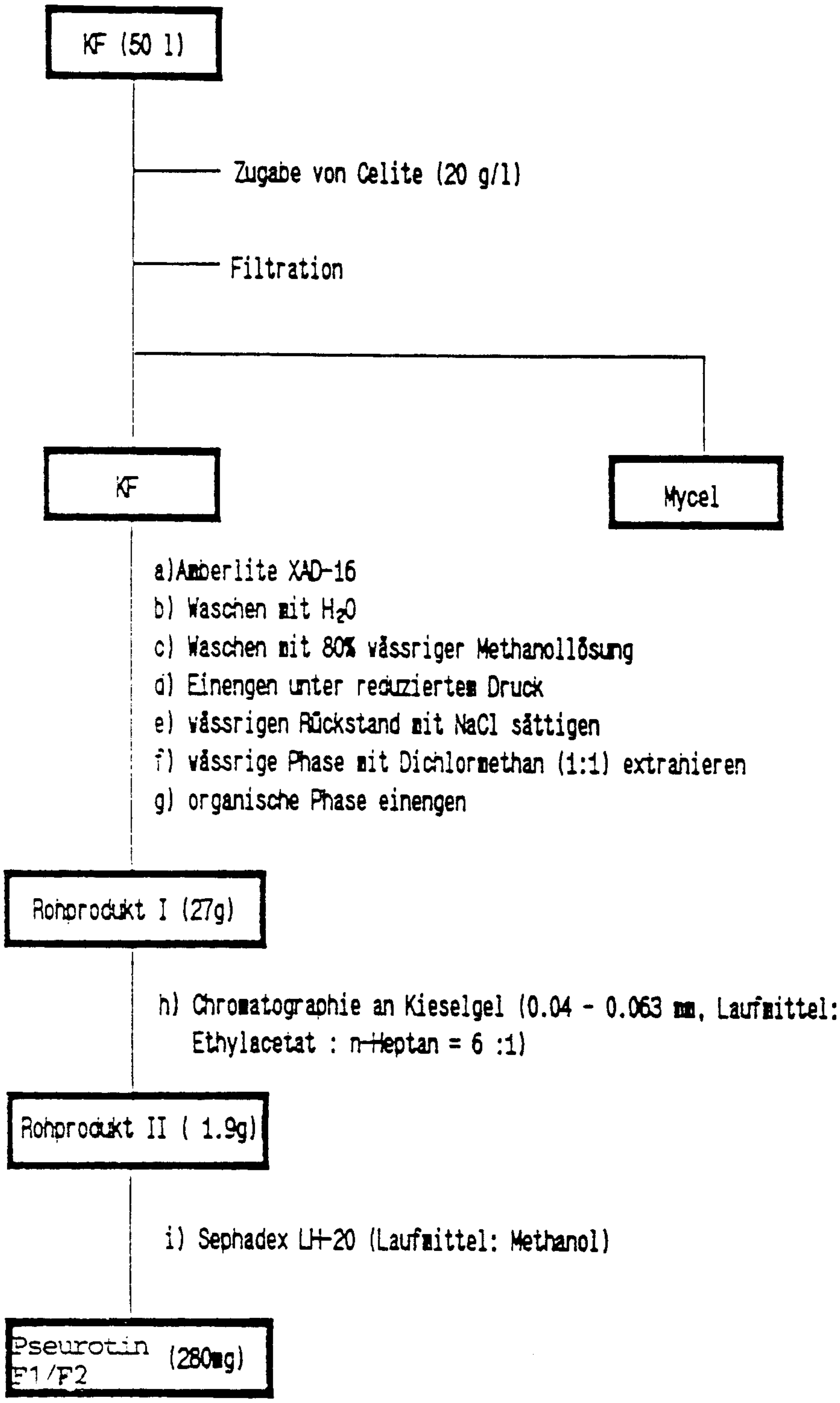


Charakterisierung der Verbindungen der Formel I:

Die Pseurotine $F_1$ /$F_2$ werden als amorpher Feststoff isoliert und sind z.B. löslich in $CH_2Cl_2$, $CHCl_3$, Essigsäureethylester, Aceton, Ethanol und Isopropanol.

FAB-MS: m/e = 418 (37 %, $[M + H]^+$), 400 (12 %) 382 (14 %)
Pseurotin $F_1$: $R_f$ = 0,29; Ethylacetat-H/Heptan (2 : 1)
Pseurotin $F_2$: $R_f$ = 0,20; Ethylacetat-H/Heptan (2 : 1) (dünnschichtchromatographisch aufgetrennt)
Molmasse: 417, 12 ($C_{21}H_{23}NO_8$)
$^1$H-NMR (400 MHz, 44 mg in 0,6 ml $CDCl_3$)

siehe Tabelle und Abbildung
$^{13}C$-NMR (100 MHz, 44 mg in 0,6 ml $CDCl_3$)
siehe Tabelle

Tabelle 2: NMR Daten von Pseurotin $F_1/F_2$ als Gemisch

| | $^{13}C$-NMR | | $^{1}H$-NMR | |
|---|---|---|---|---|
| | $F_1$ | $F_2$ | $F_1$ | $F_2$ |
| C 2 | 165,41 | 167,32 | - | |
| C 3 | 112,95 | 112,05 | - | |
| C 4 | 198,84 | 200,04 | - | |
| C 5 | 94,93 | 92,66 | - | |
| C 6 | 188,76 | | - | |

| | $^{13}$C-NMR | | $^{1}$H-NMR | |
|---|---|---|---|---|
| | $F_1$ | $F_2$ | $F_1$ | $F_2$ |
| C 8 | 89,59 | 93,65 | - | |
| C 9 | 71,58 | 77,40 | 4,81 | 4,72 |
| C10 | 70,79 | 71,10 | 4,61 ($J_{10,11}$=3Hz) | 4,68 ($J_{10,11}$ =3Hz) |
| C11 | 71,64 | 72,50 | 4,74 ($J_{10,12}$=9Hz) | 4,80 ($J_{11,12}$ =9Hz) |
| C12 | 126,09 | 126,32 | 5,09 ($J_{12,13}$=10,5Hz) | 5,06 ($J_{12,13}$=10,5 Hz) |
| C13 | 136,47 | 136,07 | 5,51 ($J_{13,14}$=7Hz) | 5,48 ($J_{13,14}$ =7Hz) |
| C14 | 21,34 | | 2,09 ($J_{14,15}$=7Hz) | |
| C15 | 14,03 | 13,99 | 0,98 | 0,99 |
| C16 | 6,26 | 6,59 | 1,64 | 1,67 |
| C17 | 194,37 | 194,29 | - | |
| C18 | 132,90 | 133,12 | - | |
| C19/23 | 131,75 | 130,69 | 8,38 (m) | 8,28 (m) |
| C20/22 | 128,40 | 128,67 | 7,42 (m) | |
| C21 | 134,57 | | 7,46 (m) | |
| C-8 OH | - | | 6,88 | 7,14 |

<table>
<tr><th></th><th colspan="2">$^{13}C$-NMR</th><th colspan="2">$^{1}H$-NMR</th></tr>
<tr><td></td><td>$F_1$</td><td>$F_2$</td><td>$F_1$</td><td>$F_2$</td></tr>
<tr><td>C-9 OH</td><td colspan="2">-</td><td colspan="2">5,8[a]</td></tr>
<tr><td>C-10 OH</td><td colspan="2">-</td><td colspan="2">4,4[a]</td></tr>
<tr><td>C-11 OH</td><td colspan="2">-</td><td colspan="2">4,0[a]</td></tr>
<tr><td>NH</td><td colspan="2">-</td><td>8,92</td><td>9,49</td></tr>
</table>

[a] Zuordnung austauschbar

Test auf die biologische Aktivität der Verbindungen der Formel I:

Die Wirkung der Verbindungen der Formeln Ia und Ib als Apomorphin Antagonisten wird an Mäusen getestet. Hierzu wird zunächst Apomorphin als 1 %ige Lösung (Verdünnungsmittel:Tylose, z.B. Methocel, Hoechst AG, Frankfurt, Deutschland) einmalig intra peritoneal oder per os injiziert. Die Dosis ist so gewählt, daß sie bei 95 % der Mäuse für ca. 30 Minuten eine anhaltende Klettertätigkeit auslöst (1,5 mg Apomorphin/kg Maus; subcutane Injektion). Tiere, die noch vor der Apomorphingabe eine konstante Klettertätigkeit aufweisen, werden nicht im Test berücksichtigt. Pseurotin $F_1$ oder $F_2$ wird 30 bzw. 60 Min. vor Apomorphin appliziert (i.p. bzw. p.o.). Es wird viermal abgelesen: unmittelbar vor sowie 10, 20 und 30 Min nach Apomorphin-Injektion. Dabei wird das Kletterverhalten pro Maus beurteilt:
Testkriterium ist, ob eine Hemmung des durch Apomorphin verursachten Kletterns eintritt. Nach 10 Minuten konnte bereits bei der Mehrzahl der mit Pseurotin $F_1$ oder $F_2$ behandelten Mäuse beobachtet werden, daß die Tiere nur noch 1 oder 2 Pfoten an der Wand hatten. Nach 20 Minuten zeigten nahezu alle Tiere deutliche Symptome in Form der Kletterhemmung (4 Pfoten am Boden).

**Patentansprüche**

**1.** Verbindungen der Formeln

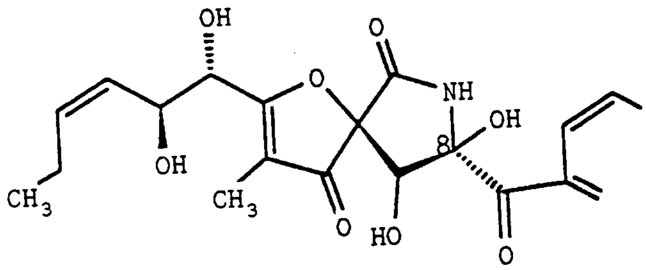

Pseurotin F1

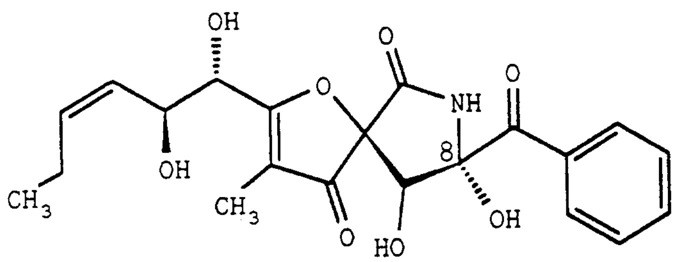

I b

Pseurotin F2

**2.** Verbindungen der Formeln Ia und Ib zur Verwendung als Arzneimittel.

**3.** Arzneimittel, enthaltend Verbindungen der Formeln Ia und Ib und pharmazeutische Träger.

**4.** Verfahren zur Herstellung der Verbindungen der Formeln Ia und Ib nach Anspruch 1, dadurch gekennzeichnet, daß Aspergillus fumigatus in einem Nährmedium kultiviert wird, bis sich die Verbindungen der Formeln Ia und Ib in der Kultur anhäufen.

**5.** Verfahren zur Herstellung der Verbindungen der Formeln Ia und Ib nach Anspruch 4, dadurch gekennzeichnet, daß Aspergillus fumigatus DSM 6598 in einem Nährmedium kultiviert wird, bis sich die Verbindungen der Formeln Ia und Ib in der Kultur anhäufen.

**6.** Verfahren nach den Ansprüchen 4 oder 5, dadurch gekennzeichnet, daß das Nährmedium 0,2 bis 5 % Malzextrakt, 0,1 bis 0,5 % Hefeextrakt, 0,5 bis 2 % Glucose und 0,01 bis 0,1 % $(NH_4)_2HPO_4$ enthält, jeweils bezogen auf das Gewicht der gesamten Nährlösung.

**7.** Verfahren nach einem oder mehreren der Ansprüche 4 bis 6, dadurch gekennzeichnet, daß die Kultivierung bei 18 bis 30 °C und in dem pH-Bereich zwischen 1 und 7 erfolgt.

**8.** Verwendung der Verbindungen der Formeln Ia und/oder Ib zur Herstellung eines Arzneimittels zur Bekämpfung von psychischen Krankheiten.

**9.** Verwendung der Verbindungen der Formeln Ia und /oder Ib als Apomorphin-Antagonist.

10. Aspergillus fumigatus DSM 6598 sowie dessen Varianten und Mutanten, soweit sie die Verbindungen der Formeln Ia und Ib nach Anspruch 1 in erhöhter Menge herstellen.

Europäisches Patentamt

# EUROPÄISCHER RECHERCHENBERICHT

Nummer der Anmeldung

EP 92 12 0724

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | HELVETICA CHIMICA ACTA<br>Bd. 64, Nr. 2, 1981, BASEL CH<br>Seiten 379 - 388<br>W. BREITENSTEIN ET AL. 'Pseurotin B, C, D and E. Further new metabolites of Pseudeutorium ovalis STOLK'<br>* Seite 380, Verbindungen 1,4 *<br>--- | 1 | C07D491/107<br>C12P17/18<br>A61K31/40<br>//(C07D491/107, 307:00,209:00)<br>(C12P17/18, C12R1:68) |
| D,A | JOURNAL OF ANTIBIOTICS.<br>Bd. 44, Nr. 4, April 1991, TOKYO JP<br>Seiten 382 - 389<br>O. ANDO ET AL. 'Synerazol, a new antifungal antibiotic'<br>* Seiten 382, 387 *<br>----- | 1-4,10 | |
| | | | **RECHERCHIERTE SACHGEBIETE (Int. Cl.5 )** |
| | | | C07D<br>C12P<br>A61K |

Der vorliegende Recherchenbericht wurde für alle Patentansprüche erstellt

| Recherchenort | Abschlußdatum der Recherche | Prüfer |
|---|---|---|
| DEN HAAG | 04 MAERZ 1993 | VOYIAZOGLOU D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)